# EUROPÄISCHE PATENTANMELDUNG

(11) **EP 2 749 322 A1**
(43) Veröffentlichungstag der Anmeldung: **02.07.2014**
(21) Anmeldenummer: 13167754.4
(22) Anmeldetag: 15.05.2013
(51) Int. Cl.: A61Q 5/08, A61Q 5/10, A61K 8/898, A61K 8/81

(54) **Verfahren zur schonenden oxidativen Haarbehandlung**

(30) Priorität: 14.06.2012 DE 102012209990
(71) Anmelder: Henkel AG&Co. KGAA, 40589 Düsseldorf (DE)
(72) Erfinder: Schulze Zur Wiesche, Erik, 20144 Hamburg (DE); Noll, Monika, 22851 Norderstedt (DE)

(57) **Zusammenfassung**

Die oxidative Haarbehandlung läßt sich schonender gestalten und insbesondere im Hinblick auf die Hydrophobisierung und starke Waschbeständigkeit verbessern, wenn ein Vorbehandlungsmittel, enthaltend aminofunktionelle(s) Silikon(e) mit terminale(r/n) Hydroxygruppe(n) auf die keratinischen Fasern aufgetragen wird, wobei das Vorbehandlungsmittel, als Spray aufgesprüht oder als Gel mit einer Viskosität unter Normalbedingungen (20°C und 1012,25 mbar) von 500 bis 5000 mPas (Brookfield RTV, Spindel 4, 20 U/min) aufgetragen wird oder als Spülung mit einer Viskosität unter Normalbedingungen (20°C und 1012,25 mbar) kleiner 500 mPas (Brookfield RTV, Spindel 4, 20 U/min) aufgetragen wird, und die keratinischen Fasern innerhalb eines Zeitraumes von einer Sekunde bis 24 Stunden nach Schritt a) mit einem oxidativen Haarbehandlungsmittel behandelt werden, wobei das oxidative Haarbehandlungsmittel eine pH Wert > 8,5 aufweist und das oxidative Haarbehandlungsmittel bezogen auf sein Gewicht mindestens 3 Gew.-% Wasserstoffperoxid (berechnet als 100%iges H₂O₂) enthält.

## Beschreibung

Die vorliegende Erfindung betrifft ein kosmetisches Verfahren zur schonenden oxidativen Haarbehandlung, bei dem keratinische Fasern vor oxidativen Einflüssen geschützt werden.

Beim Färben und beim Blondieren von Haaren tritt das Problem auf, daß es aufgrund der aggressiven Agentien zu Irritationen auf der Kopfhaut und Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die wasserabweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind sogenannte Vorbehandlungsmittel auf dem Markt, die das Haar vor dem aggressiven Einfluß schützen sollen. Diese beschweren das Haar aber oft oder beeinträchtigen den Erfolg der im Anschluß stattfindenden Aufhellung bzw. Färbung des Haares.

Der Einsatz von aminierten Silikonen in der Haarpflege ist Stand der Technik. Diese werden in Shampoos und insbesondere in Conditionern breit eingesetzt, um dort Pflegeffekte zu entfalten. So offenbart die EP 1 771 144 B1 haarkonditionierende Mittel mit aminofunktionellen Siliconen. Die dort beschriebenen Mittel sind Nachbehandlungsmittel.

Auch die europäischen Patente EP 1 312 334 B1 (Aminosilicon und Verdicker) sowie EP 1 312 335 B1 (Aminosilicon und Conditioner) offenbaren Haarnachbehandlungsmittel. Im erstgenannten Dokument werden auch extrem wasserreiche Rezepturen offenbart.

Der Einsatz von aminierten Silikonen in Vorbehandlungsmitteln zum Zwecke des Schutzes keratinischer Fasern bei einer nachfolgenden oxidativen Behandlung ist aus der EP 1 312 345 B1 bekannt. Der Einsatz 4-morpholinomethyl-substituierter Silikone in solchen Mitteln ist in der älteren deutschen Patentanmeldung 10 2011 087 344 beschrieben.

Der vorliegenden Erfindung lag die Aufgabe zugrunde, das Gebiet der Vorbehandlungsmittel zum Schutz keratinischer Fasern bei nachfolgender oxidativer Behandlung weiterzuentwickeln. Dabei sollten insbesondere die Hydrophobisierung und starke Waschbeständigkeit verbessert werden, um auch bei länger anhaltenden nachfolgenden oxidativen Behandlungen einen maximalen Schutz gewährleisten zu können.

Es wurde nun gefunden, daß eine Vorbehandlung der keratinischen Fasern insbesondere dann zu besonders gutem Schutz führt, wenn das Vorbehandlungsmittel eine bestimmte Viskosität aufweist und das im nachfolgenden oxidativen Schritt eingesetzte Mittel eine bestimmte Mindest-Oxidationsmittelmenge enthält.

Gegenstand der vorliegenden Erfindung ist in einer ersten Ausführungsform ein Verfahren zur oxidativen Haarbehandlung, bei dem
a) ein Vorbehandlungsmittel, enthaltend aminofunktionelle(s) Silikon(e) mit terminale(r/n) Hydroxygruppe(n) auf die keratinischen Fasern aufgetragen wird, wobei das Vorbehandlungsmittel,
   a als Spray aufgesprüht oder
   b als Gel mit einer Viskosität unter Normalbedingungen (20°C und 1012,25 mbar) von 500 bis 5000 mPas (Brookfield RTV, Spindel 4, 20 U/min) aufgetragen wird oder
   c als Spülung mit einer Viskosität unter Normalbedingungen (20°C und 1012,25 mbar) kleiner 500 mPas (Brookfield RTV, Spindel 4, 20 U/min) aufgetragen wird,
b) die keratinischen Fasern innerhalb eines Zeitraumes von einer Sekunde bis 24 Stunden nach Schritt a) mit einem oxidativen Haarbehandlungsmittel behandelt werden, wobei
   - das oxidative Haarbehandlungsmittel eine pH Wert > 8,5 aufweist
   - das oxidative Haarbehandlungsmittel bezogen auf sein Gewicht mindestens 3 Gew.-% Wasserstoffperoxid (berechnet als 100%iges H₂O₂) enthält.

Die im erfindungsgemäßen Verfahren eingesetzten Vorbehandlungsmittel enthalten aminofunktionelle(s) Silikon(e) mit terminale(r/n) Hydroxygruppee(n). Solche Silicone können z.B. durch die Formel

M(RₐQ_{b}SiO_{(4-a-b)/2)x}(R_{c}SiO_{(4-c)/2})_{y}M

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe mit Hydroxyfunktion ist, vorzugsweise Hydroxy-Dimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH₂-, -CH₂CH₂OCH₂-, -OCH₂CH₂-, - OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO (_{4-c})_{/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

In erfindungsgemäßen Verfahren können beispielsweise aminofunktionelle Silikone der Formel (Si-II) eingesetzt werden:

R'ₐG₃₋ₐ-Si(OSiG₂)ₙ(OSiG_{b}R'_{2-b})ₘ-O-SiG₃₋ₐ-OH (Si-II),

worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, - CH₂CH₂CH₂CH₃, -O-CH₂CH(CH₃)₂, -CH₂CH(CH₃)₂, -O-CH(CH₃)CH₂CH₃, - CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   o -Q-N(R")-CH₂-CH₂-N(R")₂
   o -Q-N(R")₂
   o -Q-N⁺(R")₃A⁻
   o -Q-N⁺H(R")₂ A⁻
   o -Q-N⁺H₂(R")A⁻
   o -Q-N(R")-CH₂CH₂N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht, R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, lodid oder Methosulfat.

In erfindungsgemäßen Verfahren dieser Ausführungsform können vorzugsweise aminofunktionelle Silikone der Formel (Si-IIa) eingesetzt werden: 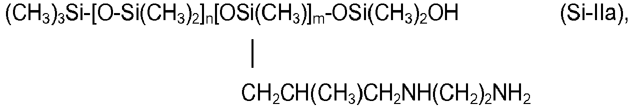 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Besonders bevorzugt sind auch erfindungsgemäße Mittel, die ein aminofunktionelles Silikon der Formel (Si-IIb) 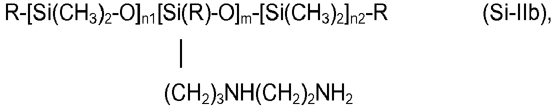 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt. Erfindungsgemäß steht dabei mindestens ein R für -OH.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Die Einsatzmenge des/der aminofunktionellen Silikon(s/e) mit terminale(r/n) Hydroxygruppee(n) in dem im erfindungsgemäßen Verfahren eingesetzten Vorbehandlungsmittel kann variieren, bevorzugte Verfahren sind dadurch gekennzeichnet, daß das Vorbehandlungsmittel bezogen auf sein Gewicht 0,00001 bis 10 Gew.-%, vorzugsweise 0,0001 bis 7,5 Gew.-%, weiter bevorzugt 0,001 bis 5 Gew.-%, noch weiter bevorzugt 0,01 bis 2,5 Gew.-% und insbesondere 0,1 bis 1 Gew.-% aminofunktionelle(s) Silikon(e) mit terminale(r/n) Hydroxygruppee(n) enthält.

Mit besonderem Vorzug ist das im erfindungsgemäßen Verfahren eingesetzte Vorbehandlungsmittel frei von Silikonen der Formeln (I) und (II) 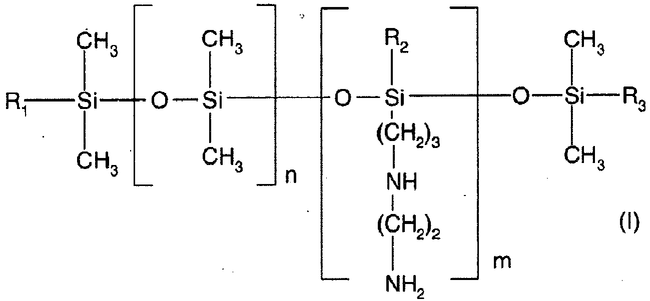 in der Formel (I):
- m und n bedeuten Zahlen, die so gewählt sind, dass die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Alkoxygruppe bedeutet; 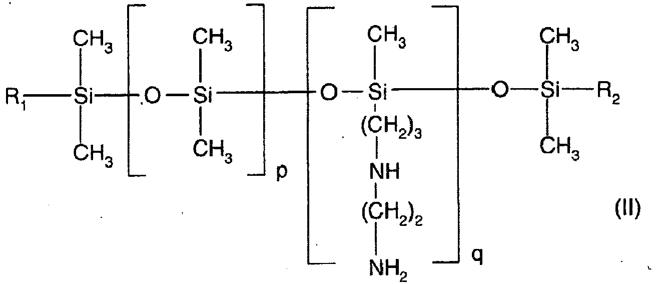 in der Formel (II):
- p und q bedeuten Zahlen, die so gewählt sind, dass die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Alkoxygruppe bedeutet.

Es hat sich herausgestellt, daß Mittel, die diese Silikone enthalten, den Erfolg der oxidativen Haarbehandlung abschwächen können, wobei gleichzeitig der Faserschutz nicht so ausgeprägt ist wie bei anderen aminofunktionellen Silikonen mit terminale(r/n) Hydroxygruppe(n).

Als besonders wirkungsvoll im Hinblick auf Faserschutz und Effektivität der nachfolgenden oxidativen Behandlung haben sich im erfindungsgemäßen Verfahren Vorbehandlungsmittel erwiesen, die mindestens ein Silikon der Formel (III) enthalten: 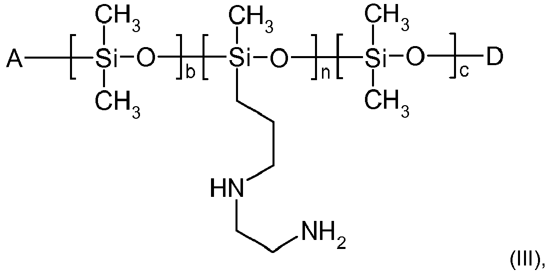 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen, mit den Maßgaben
- n > 0 und b + c > 0
- mindestens eine der Bedingungen A = -OH bzw. D = -H ist erfüllt.

In der vorstehend genannten Formel (III) sind die einzelnen Siloxaneinheiten mit den Indicse b, c und n statistisch verteilt, d.h. es muß sich nicht zwingend um Blockcopolymere handeln.

Weitere besonders geeignete Silikone sind 4-morpholinomethyl-substituiert. Erfindungsgemäße Verfahren, bei denen das Vorbehandlungsmittel mindestens ein 4-morpholinomethyl-substituiertes Silikon der Formel (IV) enthält, 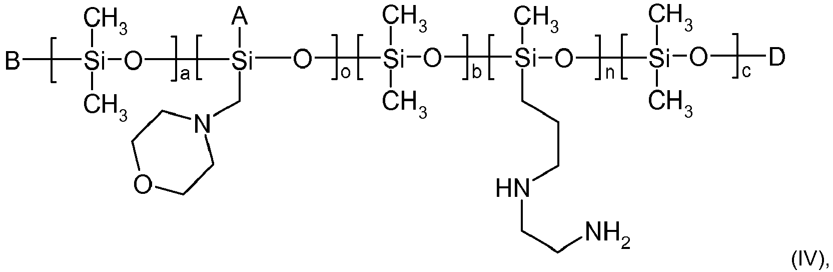 in der
- A: für eine über ein -O- gebundene Struktureinheit (i), (ii) oder (iii) 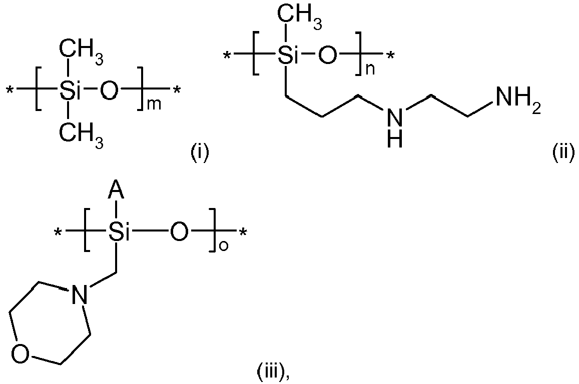 oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für-OH steht,
- *: für eine Bindung zu einer der Struktureinheiten (i), (ii) oder (iii) oder für eine Endgruppe B (Si-gebunden) oder D (O-gebunden) steht,
- B: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen.
mit der Maßgabe, daß mindestens eine der Bedingungen B = -OH bzw. D = -H erfüllt ist, sind besonders bevorzugt.

Strukturformel (IV) soll verdeutlichen, daß die Siloxangruppen n und o nicht zwingend direkt an eine Endgruppierung B bzw. D gebunden sein müssen. Vielmehr gilt in bevorzugten Formeln (IV) a > 0 oder b > 0 und in besonders bevorzugten Formeln (IV) a > 0 und b > 0, d.h. die terminale Gruppierung B bzw. D ist vorzugsweise an eine Dimethylsiloxy-Gruppierung gebunden. Auch in Formel (IV) sind die Siloxaneinheiten a, b, c, n und o vorzugsweise statistisch verteilt.

Die durch Formel (IV) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | | |
|---|---|---|
| B = -O-Si(CH₃)₂OH | und | D = -Si(CH₃)₃ |
| B = -O-Si(CH₃)₂OH | und | D = -Si(CH₃)₂OH |
| B = -O-Si(CH₃)₂OH | und | D = -Si(CH₃)₂OCH₃ |
| B = -O-Si(CH₃)₃ | und | D = -Si(CH₃)₂OH |
| B = -O-Si(CH₃)₂OCH₃ | und | D = -Si(CH₃)₂OH |

bedeutet. Diese Silikone führen zu exorbitanten Verbesserungen der Haareigenschaften der mit den erfindungsgemäßen Mitteln behandelten Haare, insbesondere zu einem gravierend verbesserten Schutz bei oxidativer Behandlung.

Auch in Formel (IV) kann der Rest A
- für eine über ein -O- gebundene Struktureinheit (i), (ii) oder (iii) oder
- einen über ein -O- gebundenen oligomeren oder polymeren Rest enthaltend Struktureinheiten der Formeln (i), (ii) oder (iii)
- oder die Hälfte eines verbindenden O-Atoms zu einer Struktureinheit (iii) oder für -OH stehen.

Damit wird Formel (IV) präzisiert zu einer der Formeln (IVa), (IVb), (IVc), (IVd), (IVe) oder (IVf):

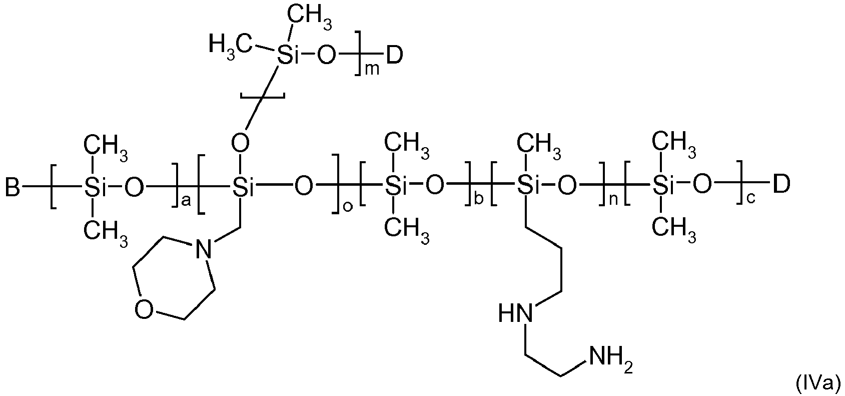

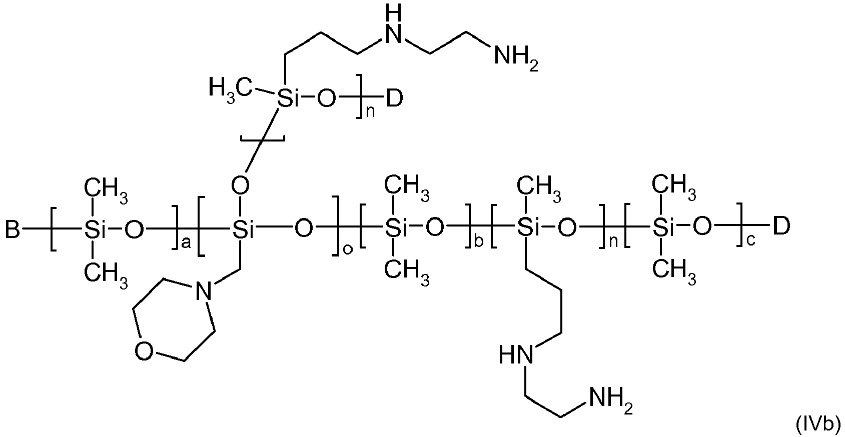

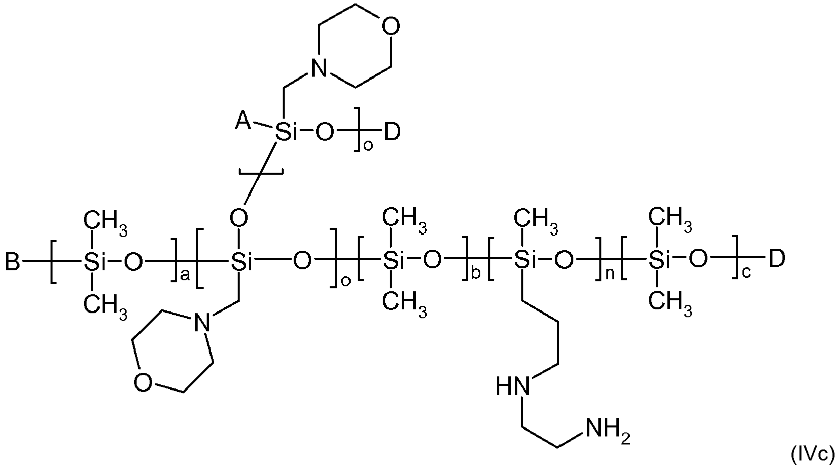

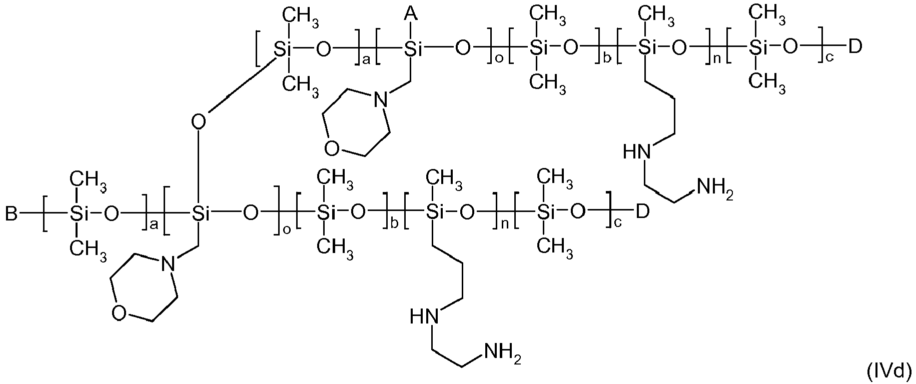

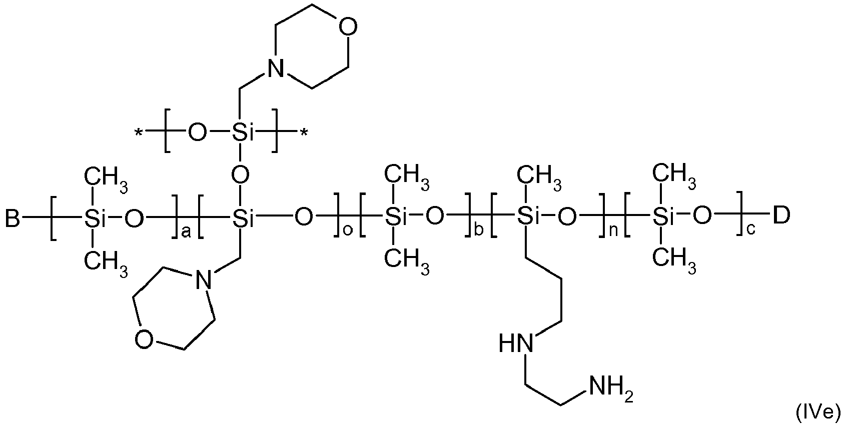

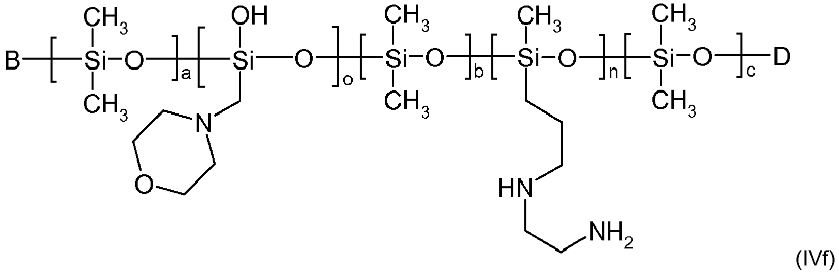

Die Struktureinheit (iii) bzw. die Siloxaneinheiten o in den Formeln (IV) können über die Gruppe A Nest- bzw. Teilkäfigstrukturen ausbilden, wenn A für die Hälfte eines verbindenden O-Atoms zu einer Struktureinheit (III) steht. Erfindungsgemäße Vorbehandlungsmittel, die Silikone mit entsprechenden 4-morpholinomethyl-substituierten Silsesquioxan-Teilstrukturen beinhalten, sind erfindungsgemäß bevorzugt, da diese Silikone zu enorm verbessertem Haarschutz vor oxidativer Behandlung führen.

Es ist in erfindungsgemäßen Verfahren der vorstehend genannten Ausführungsform besonders bevorzugt, wenn das Vorbehandlungsmittel es - bezogen auf sein Gewicht - 0,00001 bis 10 Gew.-%, vorzugsweise 0,0001 bis 7,5 Gew.-%, besonders bevorzugt 0,001 bis 5 Gew.-%, weiter bevorzugt 0,01 bis 3 Gew.-% und insbesondere 0,1 bis 1 Gew.-% 4-morpholinomethyl-substituierte(s) Silikon(e) enthält.

Unabhängig von der Art des bzw. der eingesetzten aminofunktionellen Silikon(s/e) mit terminale(r/n) Hydroxygruppee(n) enthalten die im erfindungsgemäßen Verfahren eingesetzten Vorbehandlungsmittel das/die Silikon(e) vorzugsweise in Form einer Emulsion, besonders bevorzugt in Form einer Mikroemulsion. Als besonders bevorzugt haben sich Mikroemulsionen erwiesen, die Fettalkohole als Emulgatoren bzw. Stabilisatoren enthalten, so daß bevorzugte erfindungsgemäße Verfahren dadurch gekennzeichnet sind, daß das Vorbehandlungsmittel in Form einer Mikroemulsion vorliegt, welche Fettalkohol(e) enthält.

Es hat sich gezeigt, daß die Wirkung der im Rahmen des erfindungsgemäßen Verfahrens in den Vorbehandlungsmitteln eingesetzten Silikone noch gesteigert werden kann, wenn bestimmte nichtionische Komponenten ebenfalls in den Vorbehandlungsmitteln eingesetzt werden. Zudem haben diese nichtionischen Komponenten positive Effekte auf die Lagerstabilität der Vorbehandlungsmittel. Nichtionische Komponenten, die hier besonders geeignet sind, sind Ethoxylate von Decanol, Undecanol, Dodecanol, Tridecanol usw.. Als besonders geeignet haben sich ethoxylierte Tridecanole erwiesen, die mit besonderem Vorzug in die erfindungsgemäß eingesetzten Mittel inkorporiert werden. Erfindungsgemäß besonders bevorzugt eingesetzte Vorbehandlungsmittel enthalten - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-1 0) oder deren Mischungen.

Die im erfindungsgemäßen Verfahren eingesetzten Vorbehandlungsmittel können darüber hinaus weitere übliche Inhaltsstoffe kosmetischer Mittel enthalten.

Die im erfindungsgemäßen Verfahren eingesetzten Vorbehandlungsmittel können unterschiedlich konfektioniert und appliziert werden, wobei sich neben der Applikation als Spray, die Applikation als Gel oder die Applikation als Spülung bewährt haben. Unabhängig von der Applikationsform ist eine gleichmäßige und möglichst intensive Benetzung der Haaroberfläche aller später oxidativ zu behandelnden Fasern wünschenswert.

Wird das im erfindungsgemäßen Verfahren eingesetzte Vorbehandlungsmittel als Spray aufgesprüht, so ist die kosmetische Zubereitung vorzugsweise möglichst dünnflüssig und gut versprühbar. Der große Vorteil einer solchen Applikationsform liegt darin, dass das Vorbehandlungsmittel aus dem Sprühventil als feiner Sprühnebel heraustritt, der sich in jedem Bereich des Haares niederschlägt. Hierzu ist es von Vorteil, wenn das Vorbehandlungsmittel eine Viskosität von maximal 3000 mPas, bevorzugt von maximal 2750 mPas und insbesondere von maximal 2500 mPas aufweist (gemessen mit einem Brookfield-Viskosimeter DV-II, Spindel 4 bei 20 UpM (20 s) und bei 20°C).

Eine besonders bevorzugte Applikationsform ist eine Non-Aerosol Sprühlotion. Hierbei wird das im erfindungsgemäßen Verfahren eingesetzte Vorbehandlungsmittel mit Hilfe einer geeigneten mechanisch betriebenen Sprühvorrichtung versprüht. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

In einer besonders bevorzugten Ausführungsform liegt das erfindungsgemäße Mittel in Form einer wäßrigen, alkoholischen oder wäßrig-alkoholischen Non-Aerosol Sprühlotion in Kombination mit einer geeigneten mechanischen Vorrichtung zum Versprühen oder in Form einer wäßrigen, alkoholischen oder wäßrig-alkoholischen Lotion vor.

Alternativ hierzu kann das im erfindungsgemäßen Verfahren eingesetzte Vorbehandlungsmittel als Gel mit einer Viskosität unter Normalbedingungen (20°C und 1012,25 mbar) von 500 bis 5000 mPas (Brookfield RTV, Spindel 4, 20 U/min) aufgetragen werden.

Diese Applikationsform hat den Vorteil, daß die Anwenderin das Vorbehandlungsmittel ohne Tropfen oder Kleckern gut dosieren und in die Haare einmassieren kann.

Als dritte Alternative kann das im erfindungsgemäßen Verfahren eingesetzte Vorbehandlungsmittel als Spülung mit einer Viskosität unter Normalbedingungen (20°C und 1012,25 mbar) kleiner 500 mPas (Brookfield RTV, Spindel 4, 20 U/min) aufgetragen werden. Solche dünnflüssigen Zubereitungen ermöglichen eine schnelle und sehr umfassende Benetzung aller später zu behandelnden Haaroberflächen.

Es ist für die beiden letztgenannten Varianten des erfindungsgemäßen Verfahrens vorteilhaft, wenn das Vorbehandlungsmittel bei Normalbedingungen (20°C, 1013,25 mbar) eine Viskosität (Brookfield RTV, Spindel 4, 20 U/min) von 1 bis 500 mPas (für die Spülung) bzw. von 500 bis 5000 mPas (für das Gel) aufweist. Mittel, die eine geringere Viskosität aufweisen, haben deutlich verschlechterten Haarschutz zur Folge, bei Mitteln mit höherer Viskosität besteht das Problem, daß die nachfolgende oxidative Behandlung eine deutlich schlechtere Gleichmäßigkeit erzielt, so daß ein "scheckiges" Ergebnis befürchtet werden muß.

Die Einstellung der gewünschten Viskosität kann beispielsweise mit Hilfe von Salzen, Fettstoffen oder Polymeren erfolgen, bewährt haben sich z.B. die unter der INCI-Bezeichnung Carbomer bekannten Polymere, beispielsweise ganz oder teilweise neutralisierte Polyacrylsäuren.

Das erfindungsgemäße Verfahren umfaßt das Aufbringen eines Vorbehandlungsmittels auf keratinische Fasern und eine sich daran anschließende oxidative Behandlung. Eine oxidative Haarbehandlung kann der Farb- und/oder Formveränderung der keratinischen Fasern dienen. Im Falle der Formveränderung (Dauerwelle, Glätten) wird das Haar vor der oxidativen Behandlung (Fixierung) mit einem weiteren Mittel behandelt, um die Faserstruktur formbar zu machen. In erfindungsgemäß bevorzugten Verfahren dient die oxidative Behandlung der Farbveränderung, stellt also eine Aufhellung ("Blondierung") oder ein Färben dar.

Bei der nachfolgenden oxidativen Behandlung ist es unerläßlich, daß das oxidative Haarbehandlungsmittel bezogen auf sein Gewicht mindestens 3 Gew.-% Wasserstoffperoxid (berechnet als 100%iges H₂O₂) enthält. Durch diese Mindestkonzentration wird ein gleichmäßiges und gutes Ergebnis der oxidativen Behandlung erzielt. Niedriger konzentrierte Mittel ergeben ungleichmäßige Ergebnisse und überraschenderweise - trotz geringeren oxidativen Potentials - eine höhere Haarschädigung, die sich u.a. in höheren Kontaktwinkeln zeigt.

Die im erfindungsgemäßen Verfahren eingesetzten oxidativen Haarbehandlungsmittel können neben Wasserstoffperoxid weitere Inhaltsstoffe enthalten.

Blondierprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des die in Schritt b) eingesetzten Mittels zwischen 7 und 11, insbesondere zwischen 8 und 10,5 , liegt.

Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe Ammoniak, basischen Aminosäuren, Alkalihydroxiden, Alkanolaminen, Alkalimetallmetasilikaten, Alkaliphosphaten und Alkalihydrogenphosphaten ausgewählt werden. Als Alkalimetallionen dienen bevorzugt Lithium, Natrium, Kalium, insbesondere Natrium oder Kalium.

Die als Alkalisierungsmittel einsetzbaren basischen Aminosäuren werden bevorzugt ausgewählt aus der Gruppe L-Arginin, D-Arginin, D,L-Arginin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als Alkalisierungsmittel einsetzbaren Alkalihydroxide werden bevorzugt ausgewählt aus der Gruppe Natriumhydroxid und Kaliumhydroxid.

Die als Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Alternativ kann das erfindungsgemäße Verfahren auch bei der Haarfärbung eingesetzt werden. In solchen bevorzugten Ausführungsformen ist das erfindungsgemäße Verfahren dadurch gekennzeichnet, daß Schritt b) das Aufbringen eines Haarfärbemittels umfaßt, welches zusätzlich mindestens ein Oxidationsfarbstoffvorprodukt enthält.

Als ersten zwingenden Inhaltsstoff enthalten die in Schritt b) dieses erfindungsgemäßen Verfahrens eingesetzten Mittel daher mindestens ein Oxidationsfarbstoffvorprodukt. Oxidationsfarbstoffvorprodukte können aufgrund ihres Reaktionsverhaltens in zwei Kategorien eingeteilt werden, sogenannte Entwicklerkomponenten und Kupplerkomponenten.

Entwicklerkomponenten können mit sich selbst den eigentlichen Farbstoff ausbilden. Sie können daher als alleinige, farbverändernde Verbindungen im erfindungsgemäßen Mittel enthalten sein. In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Besonders bevorzugt sind p-Phenylendiaminderivate der Formel (E1) 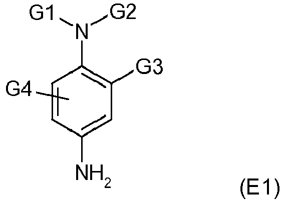 wobei
- G1 steht für ein Wasserstoffatom, einen C₁-C₄-Alkylrest, einen C₁-C₄-Monohydroxyalkylrest, einen C₂-C₄-Polyhydroxyalkylrest, einen C₁-C₄-Alkoxy-C₁-C₄-alkylrest, einen 4'-Aminophenylrest oder einen C₁-C₄-Alkylrest, der mit einer stickstoffhaltigen Gruppe, einem Phenyl- oder einem 4'-Aminophenylrest substituiert ist;
- G2 steht für ein Wasserstoffatom, einen C₁-C₄-Alkylrest, einen C₁-C₄-Monohydroxyalkylrest, einen C₂-C₄-Polyhydroxyalkylrest, einen C₁-C₄-Alkoxy-C₁-C₄-alkylrest oder einen C₁-C₄-Alkylrest, der mit einer stickstoffhaltigen Gruppe substituiert ist;
- G3 steht für ein Wasserstoffatom, ein Halogenatom, wie ein Chlor-, Brom-, lod- oder Fluoratom, einen C₁-C₄-Alkylrest, einen C₁-C₄-Monohydroxyalkylrest, einen C₂-C₄-Polyhydroxyalkylrest, einen C₁-C₄-Hydroxyalkoxyrest, einen C₁-C₄-Alkoxy-C₁-C₄-alkylrest, einen C₁-C₄-Acetylaminoalkoxyrest, einen Mesylamino-C₁-C₄-alkoxyrest oder einen C₁-C₄-Carbamoyl-aminoalkoxyrest;
- G4 steht für ein Wasserstoffatom, ein Halogenatom, einen C₁-C₄-Alkylrest oder einen C₁-C₄-Alkoxy-C₁-C₄-alkylrest oder
- wenn G3 und G4 in ortho-Stellung zueinander stehen, können sie gemeinsam eine verbrückende α,ω-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus 1,4-Diaminobenzol (p-Phenylendiamin), 1,4-Diamino-2-methylbenzol (p-Toluylendiamin), 1,4-Diamino-2-chlorbenzol (2-Chlor-p-phenylendiamin), 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)amino-2-methylanilin, 4-N,N-Bis-(2-hydroxyethyl)amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1 H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 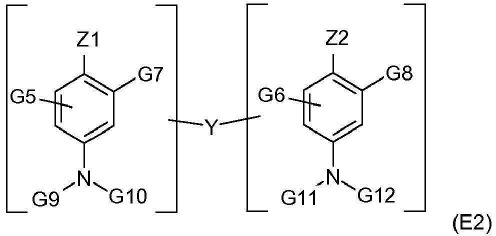 wobei
- Z1 und Z2 stehen unabhängig voneinander für einen Hydroxyl- oder NH₂-Rest, der gegebenenfalls durch einen C₁-C₄-Alkylrest, durch einen C₁-C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G5 und G6 stehen unabhängig voneinander für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Aminoalkylrest oder eine direkte Verbindung zur Verbrückung Y,
- G7, G8, G9, G10, G11 und G12 stehen unabhängig voneinander für ein Wasserstoffatom, eine direkte Bindung zur Verbrückung Y oder einen C₁-C₄-Alkylrest,
mit der Maßgabe, dass die Verbindungen der Formel (E2) nur eine Verbrückung Y pro Molekül enthalten.

Bevorzugte zweikernige Entwicklerkomponenten der Formel (E2) werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)tetramethylendiamin, N,N'-Bis-(4-(methylamino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 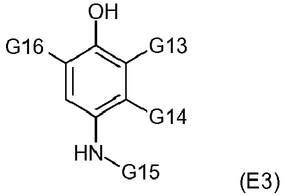 wobei
- G13 steht für ein Wasserstoffatom, ein Halogenatom, einen C₁-C₄-Alkylrest, einen C₁-C₄-Mono-hydroxyalkylrest, einen C₂-C₄-Polyhydroxyalkylrest, einen C₁-C₄-Alkoxy-C₁-C₄-alkylrest, einen C₁-C₄-Aminoalkylrest, einen Hydroxy-C₁-C₄-alkylaminorest, einen C₁-C₄-Hydroxyalkoxyrest, einen C₁-C₄-Hydroxyalkyl-C₁-C₄-aminoalkylrest oder einen Di-(C₁-C₄-alkyl)amino-C₁-C₄-alkylrest, und
- G14 steht für ein Wasserstoff- oder Halogenatom, einen C₁-C₄-Alkylrest, einen C₁-C₄-Mono-hydroxyalkylrest, einen C₂-C₄-Polyhydroxyalkylrest, einen C₁-C₄-Alkoxy-C₁-C₄-alkylrest, einen C₁-C₄-Aminoalkylrest oder einen C₁-C₄-Cyanoalkylrest,
- G15 steht für Wasserstoff, einen C₁-C₄-Alkylrest, einen C₁-C₄-Monohydroxyalkylrest, einen C₂-C₄-Polyhydroxyalkylrest, einen Phenylrest oder einen Benzylrest, und
- G16 steht für Wasserstoff oder ein Halogenatom.

Bevorzugte p-Aminophenole der Formel (E3) sind insbesondere 4-Aminophenol, N-Methyl-4-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-amino-methylphenol, 4-Amino-2-(2-hydroxyethyl-aminomethyl)phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze.

Ganz besonders bevorzugte Verbindungen der Formel (E3) sind 4-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Bevorzugte Pyrimidinderivate werden erfindungsgemäß ausgewählt aus Verbindungen gemäß Formel (E4) bzw. deren physiologisch verträglichen Salzen, 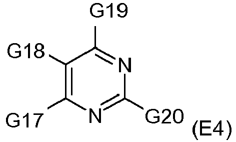 worin
- G17, G18 und G19 unabhängig voneinander für ein Wasserstoffatom, eine Hydroxygruppe, eine C₁-C₄-Alkoxygruppe oder eine Aminogruppe steht und
- G20 für eine Hydroxygruppe oder eine Gruppe -NG21G22 steht, worin G21 und G22 unabhängig voneinander stehen für ein Wasserstoffatom, eine C₁-C₄-Alkylgruppe, eine C₁-C₄-Mono-hydroxyalkylgruppe,
mit der Maßgabe, dass maximal zwei der Gruppen aus G17, G18, G19 und G20 eine Hydroxygruppe bedeuten und höchstens zwei der Reste G17, G18 und G19 für ein Wasserstoffatom stehen. Dabei ist es wiederum bevorzugt, wenn gemäß Formel (E4) mindestens zwei Gruppen aus G17, G18, G19 und G20 für eine Gruppe -NG21G22 stehen und höchstens zwei Gruppen aus G17, G18, G19 und G20 für eine Hydroxygruppe stehen.

Besonders bevorzugte Pyrimidinderivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazolderivate werden erfindungsgemäß ausgewählt aus Verbindungen gemäß Formel (E5), 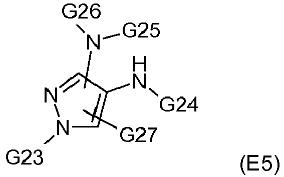 worin
- G23, G24, G25 stehen unabhängig voneinander für ein Wasserstoffatom, eine C₁-C₄-Alkylgruppe, eine C₁-C₄-Monohydroxyalkylgruppe, eine C₂-C₄-Polyhydroxyalkylgruppe, eine gegebenenfalls substituierte Arylgruppe oder eine gegebenenfalls substituierte Aryl-C₁-C₄-alkylgruppe, mit der Maßgabe dass, wenn G25 für ein Wasserstoffatom steht, G26 neben den vorgenannten Gruppen zusätzlich für eine Gruppe -NH₂ stehen kann,
- G26 steht für ein Wasserstoffatom, eine C₁-C₄-Alkylgruppe, eine C₁-C₄-Monohydroxyalkyl-gruppe oder eine C₂-C₄-Polyhydroxyalkylgruppe und
- G27 steht für ein Wasserstoffatom, eine gegebenenfalls substituierte Arylgruppe, eine C₁-C₄-Alkylgruppe oder eine C₁-C₄-Mononohydroxyalkylgruppe, insbesondere für ein Wasserstoffatom oder eine Methylgruppe.

Bevorzugt bindet in Formel (E5) der Rest -NG25G26 an die 5 Position und der Rest G27 an die 3-Position des Pyrazolcyclus.

Besonders bevorzugte Pyrazolderivate sind insbesondere die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazino-pyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)-amino-1,3-dimethylpyrazol, sowie deren physiologisch verträglichen Salze, insbesondere jedoch 4,5-Diamino-1-(2-hydroxyethyl)pyrazol.

Bevorzugte Pyrazolopyrimidin-Derivate sind insbesondere die Derivate des Pyrazolo[1,5-a]-pyrimidin der folgenden Formel (E6) und dessen tautomeren Formen, sofern ein tautomeres Gleichgewicht besteht: 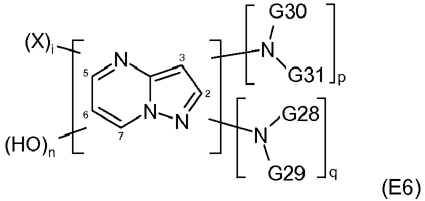 wobei:
- G28, G29 und G30, G31 unabhängig voneinander stehen für ein Wasserstoffatom, einen C₁-C₄-Alkylrest, einen Aryl-Rest, einen C₁-C₄-Monohydroxyalkylrest, einen C₂-C₄-Polyhydroxyalkylrest, einen C₁-C₄-Alkoxy-C₁-C₄-alkylrest, einen C₁-C₄-Aminoalkylrest, der gegebenenfalls durch ein Acetyl-Ureid- oder einen Sulfonyl-Rest geschützt sein kann, einen C₁-C₄-Alkylamino-C₁-C₄-alkylrest, einen Di-(C₁-C₄-alkyl)amino-C₁-C₄-alkylrest, wobei die Dialkyl-Reste gegebenenfalls einen Kohlenstoffcyclus oder einen Heterocyclus mit 5 oder 6 Kettengliedern bilden, einen C₁-C₄-Monohydroxyalkyl- oder einen Bis-(C₁-C₄-Hydroxyalkyl)amino-C₁-C₄-alkylrest,
- die X-Reste stehen unabhängig voneinander für ein Wasserstoffatom, einen C₁-C₄-Alkylrest, einen Aryl-Rest, einen C₁-C₄-Monohydroxyalkylrest, einen C₂-C₄-Polyhydroxyalkylrest, einen C₁-C₄-Aminoalkylrest, einen C₁-C₄-Alkylamino-C₁-C₄-alkylrest, einen Di-(C₁-C₄-alkyl)amino-C₁-C₄-alkylrest, wobei die Dialkyl-Reste gegebenenfalls einen Kohlenstoffcyclus oder einen Heterocyclus mit 5 oder 6 Kettengliedern bilden, einen C₁-C₄-Hydroxyalkyl- oder einen Di-(C₁-C₄-Hydroxyalkyl)amino-C₁-C₄-alkylrest, einen Aminorest, einen C₁-C₄-Alkylaminorest, einen Di-(C₁-C₄-alkyl)aminorest, C₁-C₄-hydroxyalkylaminorest oder einen Di-(C₁-C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG28G29 und NG30G31 belegen die Positionen (2,3); (5,6); (6,7); (3,5) oder (3,7);
- wenn p + q gleich 1 ist, n den Wert 1 hat, und die Gruppen NG28G29 (oder NG30G31) und die Gruppe OH belegen die Positionen (2,3); (5,6); (6,7); (3,5) oder (3,7);

Wenn das Pyrazolo[1,5-a]pyrimidin der oben stehenden Formel (E6) eine Hydroxygruppe an einer der Positionen 2, 5 oder 7 des Ringsystems enthält, besteht ein tautomeres Gleichgewicht, das zum Beispiel im folgenden Schema dargestellt wird:

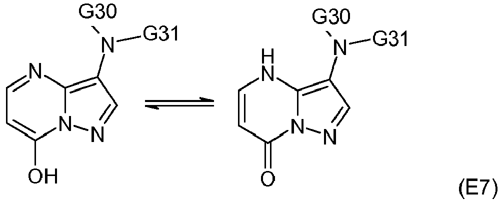

Unter den Pyrazolo[1,5-a]pyrimidinen der obenstehenden Formel (E7) kann man insbesondere nennen: Pyrazolo[1,5-a]pyrimidin-3,7-diamin, 2,5-Dimethylpyrazolo[1,5-a]pyrimidin-3,7-diamin, Pyrazolo[1,5-a]pyrimidin-3,5-diamin, 2,7-Dimethylpyrazolo[1,5-a]pyrimidin-3,5-diamin, 3-Amino-pyrazolo[1,5-a]pyrimidin-7-ol, 3-Aminopyrazolo[1,5-a]pyrimidin-5-ol, 2-(3-Aminopyrazolo[1,5-a]-pyrimidin-7-ylamino)ethanol, 2-(7-Aminopyrazolo[1,5-a]pyrimidin-3-ylamino)ethanol, 2-[(3-Amino-pyrazolo[1,5-a]pyrimidin-7-yl)-(2-hydroxyethyl)amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist.

Im Folgenden werden Beispiele für die als Substituenten der Verbindungen der Formeln (E1) bis (E6) genannten Reste aufgezählt: Beispiele für C₁-C₄-Alkylreste sind die Gruppen CH₃, CH₂CH₃, CH₂CH₂CH₃, CH(CH₃)₂, CH₂CH₂CH₂CH₃, CH₂CH(CH₃)₂, CH(CH₃)CH₂CH₃, C(CH₃)₃. Erfindungsgemäße Beispiele für C₁-C₄-Alkoxyreste sind OCH₃, OCH₂CH₃, OCH₂CH₂CH₃, OCH(CH₃)₂, OCH₂CH₂CH₂CH₃, OCH₂CH(CH₃)₂, OCH(CH₃)CH₂CH₃, OC(CH₃)₃, insbesondere eine Methoxy- oder eine Ethoxygruppe.

Weiterhin sind bevorzugte Beispiele für eine C₁-C₄-Monohydroxyalkylgruppe CH₂OH, CH₂CH₂OH, CH₂CH₂CH₂OH, CHCH(OH)CH₃, CH₂CH₂CH₂CH₂OH, wobei die Gruppe CH₂CH₂OH bevorzugt ist. Ein besonders bevorzugtes Beispiel einer C₂-C₄-Polyhydroxyalkylgruppe ist die 1,2-Dihydroxyethylgruppe.

Beispiele für Halogenatome sind F-, Cl- oder Br-Atome, Cl-Atome sind ganz besonders bevorzugte Beispiele.

Beispiele für stickstoffhaltige Gruppen sind insbesondere NH₂, C₁-C₄-Monoalkylaminogruppen, Di-(C₁-C₄-alkyl)aminogruppen, Tri-(C₁-C₄-alkyl)ammoniumgruppen, C₁-C₄-Monohydroxy-alkylaminogruppen, Imidazolinium und NH₃⁺.

Beispiele für C₁-C₄-Monoalkylaminogruppen sind NHCH₃, NHCH₂CH₃, NHCH₂CH₂CH₃, NHCH(CH₃)₂.

Beispiele für Di-(C₁-C₄-alkyl)aminogruppe sind N(CH₃)₂, N(CH₂CH₃)₂.

Beispiele für Tri-(C₁-C₄-alkyl)ammoniumgruppen sind N⁺(CH₃)₃, N⁺(CH₃)₂(CH₂CH₃), N⁺(CH₃)(CH₂CH₃)₂.

Beispiele für C₁-C₄-Hydroxyalkylaminoreste sind NHCH₂CH₂OH, NHCH₂CH₂OH, NHCH₂CH₂CH₂OH, NHCH₂CH₂CH₂OH.

Beispiele für C₁-C₄-Alkoxy-C₁-C₄-alkylgruppen sind die Gruppen CH₂CH₂OCH₃, CH₂CH₂CH₂OCH₃, CH₂CH₂OCH₂CH₃, CH₂CH₂CH₂OCH₂CH₃, CH₂CH₂OCH(CH₃)₂, CH₂CH₂CH₂OCH(CH₃)₂.

Beispiele für Hydroxy-C₁-C₄-alkoxyreste sind OCH₂OH, OCH₂CH₂OH, OCH₂CH₂CH₂OH, OCH₂CH(OH)CH₃, OCH₂CH₂CH₂CH₂OH.

Beispiele für C₁-C₄-Acetylaminoalkoxyreste sind OCH₂NHC(O)CH₃, OCH₂CH₂NHC(O)CH₃, OCH₂CH₂CH₂NHC(O)CH3, OCH₂CH(NHC(O)CH₃)CH₃, OCH₂CH₂CH₂CH₂NHC(O)CH₃.

Beispiele für C₁-C₄-Carbamoylaminoalkoxyreste sind OCH₂CH₂NHC(O)NH₂, OCH₂CH₂CH₂NHC(O)NH₂, OCH₂CH₂CH₂CH₂NHC(O)NH₂.

Beispiele für C₁-C₄-Aminoalkylreste sind CH₂NH₂, CH₂CH₂NH₂, CH₂CH₂CH₂NH₂, CH₂CH(NH₂)CH₃, CH₂CH₂CH₂CH₂NH₂.

Beispiele für C₁-C₄-Cyanoalkylreste sind CH₂CN, CH₂CH₂CN, CH₂CH₂CH₂CN.

Beispiele für C₁-C₄-Hydroxyalkylamino-C₁-C₄-alkylreste sind CH₂CH₂NHCH₂CH₂OH, CH₂CH₂CH₂NHCH₂CH₂OH, CH₂CH₂NHCH₂CH₂CH₂OH, CH₂CH₂CH₂NHCH₂CH₂CH₂OH.

Beispiele für Di-(C₁-C₄-Hydroxyalkyl)amino-C₁-C₄-alkylreste sind CH₂CH₂N(CH₂CH₂OH)₂, CH₂CH₂CH₂N(CH₂CH₂OH)₂, CH₂CH₂N(CH₂CH₂CH₂OH)₂, CH₂CH₂CH₂N(CH₂CH₂CH₂OH)₂.

Ein Beispiel für eine Arylgruppe ist die Phenylgruppe.

Beispiele für Aryl-C₁-C₄-alkylgruppen sind die Benzylgruppe und die 2-Phenylethylgruppe. Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxy-ethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Ganz besonders bevorzugte Entwicklerkomponenten sind dabei p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze.

Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt cyclische Verbindungen, die am Cyclus mindestens zwei Gruppen tragen, ausgewählt aus (i) gegebenenfalls substituierten Aminogruppen und/oder (ii) Hydroxylgruppen. Wenn die cyclische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:
- 3-Aminophenol (m-Aminophenol) und/oder dessen Derivate,
- 3-Aminoanilin (m-Diaminobenzol) und/oder dessen Derivate,
- 2-Aminoanilin (1,2-Diaminobenzol; o-Diaminobenzol) und/oder dessen Derivate,
- 2-Aminophenol (o-Aminophenol) und/oder dessen Derivate,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin,

Gemische aus zwei oder mehreren Verbindungen aus einer oder mehreren dieser Klassen sind im Rahmen dieser Ausführungsform ebenso erfindungsgemäß.

Die erfindungsgemäß verwendbaren 3-Aminophenole (m-Aminophenole) bzw. deren Derivate werden bevorzugt ausgewählt aus mindestens einer Verbindung der Formel (K1) und/oder aus mindestens einem physiologisch verträglichen Salz einer Verbindung gemäß Formel (K1), 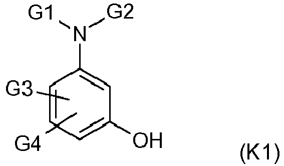 worin
- G1 und G2: unabhängig voneinander stehen für ein Wasserstoffatom, eine C₁-C₄-Alkylgruppe, eine C₃-C₆-Cycloalkylgruppe, eine C₂-C₄-Alkenylgruppe, eine C₁-C₄-Monohydroxy-alkylgruppe, eine C₂-C₄-Polyhydroxyalkylgruppe, eine C₂-C₄-Perfluoracylgruppe, eine Aryl-C₁-C₆-alkylgruppe, eine Amino-C₁-C₆-alkylgruppe, eine Di-(C₁-C₆-alkyl)amino-C₁-C₆-alkylgruppe oder eine C₁-C₆-Alkoxy-C₁-C₆-alkylgruppe, wobei G1 und G2 gemeinsam mit dem Stickstoffatom einen fünfgliedrigen, sechsgliedrigen oder siebengliedrigen Ring bilden können,
- G3 und G4: unabhängig voneinander stehen für ein Wasserstoffatom, ein Halogenatom, eine C₁-C₄-Alkylgruppe, eine C₁-C₄-Alkoxygruppe, eine Hydroxygruppe, eine C₁-C₄-Mono-hydroxyalkylgruppe, eine C₂-C₄-Polyhydroxyalkylgruppe, eine Hydroxy-C₁-C₄-alkoxygruppe, eine C₁-C₆-Alkyoxy-C₂-C₆-alkoxygruppe, eine Arylgruppe oder eine Heteroarylgruppe.

Besonders bevorzugte 3-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxy-ethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren 3-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2), 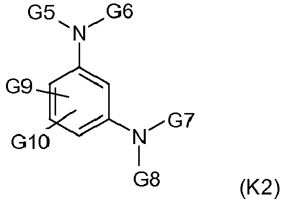 worin
- G5, G6, G7 und G8: unabhängig voneinander stehen für ein Wasserstoffatom, eine C₁-C₄-Alkylgruppe, eine C₃-C₆-Cycloalkylgruppe, eine C₂-C₄-Alkenylgruppe, eine C₁-C₄-Monohydroxyalkylgruppe, eine C₂-C₄-Polyhydroxyalkylgruppe, eine C₁-C₄-Alkoxy-C₁-C₄-alkylgruppe, eine Aryl-C₁-C₄-alkylgruppe, eine Heteroaryl-C₁-C₄-alkylgruppe, eine C₂-C₄-Perfluoracylgruppe, oder gemeinsam mit dem Stickstoffatom einen fünfgliedrigen oder sechsgliedrigen Heterocyclus bilden
- G9 und G10: unabhängig voneinander stehen für ein Wasserstoffatom, ein Halogenatom, eine C₁-C₄-Alkylgruppe, eine ω-(2,4-Diaminophenyl)-C₁-C₄-alkylgruppe, eine ω-(2,4-Diaminophenyloxy)-C₁-C₄-alkoxygruppe, eine C₁-C₄-Alkoxygruppe, eine Hydroxygruppe, eine C₁-C₄-Alkoxy-C₂-C₄-alkoxygruppe, eine Arylgruppe, eine Heteroarylgruppe, eine C₁-C₄-Monohydroxyalkylgruppe, eine C₂-C₄-Polyhydroxyalkylgruppe, eine Hydroxy-C₁-C₄-alkoxygruppe.

Besonders bevorzugte 3-Diaminobenzol-Kupplerkomponenten werden ausgewählt aus mindestens einer Verbindung aus der Gruppe, die gebildet wird aus 3-Aminoanilin (m-Phenylendiamin), 2-(2,4-Diaminophenoxy)ethanol, 1,3-Bis(2,4-diaminophenoxy)propan, 1-Methoxy-2-amino-4-(2'-hydroxy-ethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)aminobenzol und den physiologisch verträglichen Salzen aller vorstehend genannten Verbindungen.

Die erfindungsgemäß verwendbaren 1,2-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3), 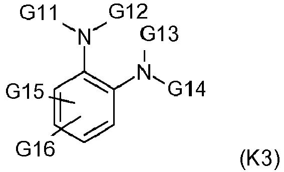 worin
- G11, G12, G13 und G14: unabhängig voneinander stehen für ein Wasserstoffatom, eine C₁-C₄-Alkylgruppe, eine C₃-C₆-Cycloalkylgruppe, eine C₂-C₄-Alkenylgruppe, eine C₁-C₄-Monohydroxyalkylgruppe, eine C₂-C₄-Polyhydroxyalkylgruppe, eine C₁-C₄-Alkoxy-C₁-C₄-alkylgruppe, eine Aryl-C₁-C₄-alkylgruppe, eine Heteroaryl-C₁-C₄-alkylgruppe, eine C₂-C₄-Perfluoracylgruppe, oder gemeinsam mit dem Stickstoffatom einen fünfgliedrigen oder sechsgliedrigen Heterocyclus bilden
- G15 und G16: unabhängig voneinander stehen für ein Wasserstoffatom, ein Halogenatom, eine Carboxylgruppe, eine C₁-C₄-Alkylgruppe, eine C₁-C₄-Alkoxygruppe, eine Hydroxygruppe, eine C₁-C₄-Monohydroxyalkylgruppe, eine C₂-C₄-Polyhydroxyalkylgruppe, eine Hydroxy-C₁-C₄-alkoxygruppe.

Besonders bevorzugte 1,2-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4), 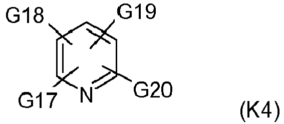 worin
- G17 und G18: stehen unabhängig voneinander für eine Hydroxygruppe oder eine Gruppe -NG21G22,
worin G21 und G22 unabhängig voneinander stehen für ein Wasserstoffatom, eine C₁-C₄-Alkylgruppe, eine C₃-C₆-Cycloalkylgruppe, eine C₂-C₄-Alkenylgruppe, eine Arylgruppe, eine C₁-C₄-Monohydroxyalkylgruppe, eine C₂-C₄-Polyhydroxyalkylgruppe, eine C₁-C₄-Alkoxy-C₁-C₄-alkylgruppe, eine Aryl-C₁-C₄-alkylgruppe, eine Heteroaryl-C₁-C₄-alkylgruppe,
- G19 und G20: stehen unabhängig voneinander für ein Wasserstoffatom, ein Halogenatom, eine C₁-C₄-Alkylgruppe oder eine C₁-C₄-Alkoxygruppe.

Es ist bevorzugt, wenn gemäß Formel (K4) die Reste G17 und G18 in ortho-Position oder in meta-Position zueinander stehen.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5), 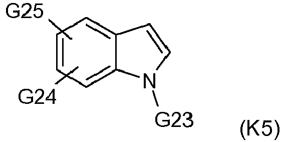 worin
- G23: steht für ein Wasserstoffatom, eine C₁-C₄-Alkylgruppe, eine C₃-C₆-Cycloalkylgruppe, eine C₂-C₄-Alkenylgruppe, eine C₁-C₄-Monohydroxyalkylgruppe, eine C₂-C₄-Polyhydroxyalkylgruppe, eine Aryl-C₁-C₄-alkylgruppe,
- G24: steht für eine Hydroxygruppe oder eine Gruppe NG26G27, worin G26 und G27 unabhängig voneinander stehen für ein Wasserstoffatom, eine C₁-C₄-Alkylgruppe, eine C₃-C₆-Cycloalkylgruppe, eine C₂-C₄-Alkenylgruppe, eine C₁-C₄-Monohydroxyalkylgruppe, eine C₂-C₄-Polyhydroxyalkylgruppe,
- G25: Wasserstoffatom, ein Halogenatom oder eine C₁-C₄-Alkylgruppe,
mit der Maßgabe, dass G24 in meta-Position oder ortho-Position zum Strukturfragment NG23 der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6), 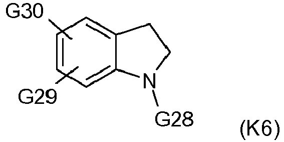 worin
- G28: steht für ein Wasserstoffatom, eine C₁-C₄-Alkylgruppe, eine C₃-C₆-Cycloalkylgruppe, eine C₂-C₄-Alkenylgruppe, eine C₁-C₄-Monohydroxyalkylgruppe, eine C₂-C₄-Polyhydroxyalkylgruppe, eine Aryl-C₁-C₄-alkylgruppe,
- G29: steht für eine Hydroxygruppe oder eine Gruppe NG31G32, worin G31 und G32 unabhängig voneinander stehen für ein Wasserstoffatom, eine C₁-C₄-Alkylgruppe, eine C₃-C₆-Cycloalkylgruppe, eine C₂-C₄-Alkenylgruppe, eine C₁-C₄-Monohydroxyalkylgruppe, eine C₂-C₄-Polyhydroxyalkylgruppe,
- G30: Wasserstoffatom, ein Halogenatom oder eine C₁-C₄-Alkylgruppe,
mit der Maßgabe, dass G29 in meta-Position oder ortho-Position zum Strukturfragment NG28 der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Im Folgenden werden Beispiele für die als Substituenten der Verbindungen der Formeln (K1) bis (K6) genannten Reste aufgezählt: Beispiele für C₁-C₄-Alkylreste sind die Gruppen CH₃, CH₂CH₃, CH₂CH₂CH₃, CH(CH₃)₂, CH₂CH₂CH₂CH₃, CH₂CH(CH₃)₂, CH(CH₃)CH₂CH₃, C(CH₃)₃. Erfindungsgemäße Beispiele für C₃-C₆-Cycloalkylgruppen sind die Cyclopropyl-, die Cyclopentyl-und die Cyclohexylgruppe.

Erfindungsgemäße Beispiele für C₁-C₄-Alkoxyreste sind OCH₃, OCH₂CH₃, OCH₂CH₂CH₃, OCH(CH₃)₂, OCH₂CH₂CH₂CH₃, OCH₂CH(CH₃)₂, OCH(CH₃)CH₂CH₃, OC(CH₃)₃, insbesondere eine Methoxy- oder eine Ethoxygruppe.

Weiterhin können als bevorzugte Beispiele für eine C₁-C₄-Monohydroxyalkylgruppe CH₂OH, CH₂CH₂OH, CH₂CH₂CH₂OH, CH₂CH(OH)CH₃, CH₂CH₂CH₂CH₂OH genannt werden, wobei die Gruppe CH₂CH₂OH bevorzugt ist.

Ein besonders bevorzugtes Beispiel einer C₂-C₄-Polyhydroxyalkylgruppe ist die 1,2-Dihydroxyethylgruppe.

Beispiele für Halogenatome sind F-, Cl- oder Br-Atome, Cl-Atome sind ganz besonders bevorzugte Beispiele.

Beispiele für stickstoffhaltige Gruppen sind insbesondere NH₂, C₁-C₄-Monoalkylaminogruppen, Di-(C₁-C₄-alkyl)aminogruppen, Tri-(C₁-C₄-alkyl)ammoniumgruppen, Mono-(C₁-C₄-hydroxyalkyl)-aminogruppen, Imidazolinium und NH₃⁺.

Beispiele für C₁-C₄-Monoalkylaminogruppen sind NHCH₃, NHCH₂CH₃, NHCH₂CH₂CH₃, NHCH(CH₃)₂.

Beispiele für eine Di-(C₁-C₄-alkyl)aminogruppe sind N(CH₃)₂, N(CH₂CH₃)₂.

Beispiele für C₁-C₄-Alkoxy-C₁-C₄-alkylgruppen sind CH₂CH₂OCH₃, CH₂CH₂CH₂OCH₃, CH₂CH₂OCH₂CH₃, CH₂CH₂CH₂OCH₂CH₃, CH₂CH₂OCH(CH₃)₂, CH₂CH₂CH₂OCH(CH₃)₂.

Beispiele für C₁-C₄-Alkoxy-C₁-C₄-alkoxygruppen sind OCH₂CH₂OCH₃, OCH₂CH₂CH₂OCH₃, OCH₂CH₂OCH₂CH₃, OCH₂CH₂CH₂OCH₂CH₃, OCH₂CH₂OCH(CH₃)₂, OCH₂CH₂CH₂OCH(CH₃)₂. Beispiele für Hydroxy-C₁-C₄-alkoxyreste sind OCH₂OH, OCH₂CH₂OH, CH₂CH₂CH₂OH, OCH₂CH(OH)CH₃, OCH₂CH₂CH₂CH₂OH.

Beispiele für C₁-C₄-Aminoalkylreste sind CH₂NH₂, CH₂CH₂NH₂, CH₂CH₂CH₂NH₂, CH₂CH(NH₂)CH₃, CH₂CH₂CH₂CH₂NH₂.

Ein Beispiel für eine Arylgruppe ist die Phenylgruppe, die auch substituiert sein kann.

Beispiele für Aryl-C₁-C₄-alkylgruppen sind die Benzylgruppe und die 2-Phenylethylgruppe. Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Ganz besonders bevorzugt ist dabei Resorcin, 2-Methylresorcin, 5-Amino-2-methylphenol, 3-Aminophenol, 2-(2,4-Diaminophenoxy)ethanol, 1,3-Bis(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Im Rahmen der vorliegenden Erfindung sind folgende Kombinationen aus Oxidationsfarbstoffvorprodukten vom Entwicklertyp und vom Kupplertyp besonders bevorzugt Mit den als Kombination genannten Oxidationsfarbstoffvorprodukten können jedoch auch noch weitere Farbstoffvorprodukte kombiniert werden:

p-Toluylendiamin / Resorcin; p-Toluylendiamin / 2-Methylresorcin; p-Toluylendiamin / 5-Amino-2-methylphenol; p-Toluylendiamin / 3-Aminophenol; p-Toluylendiamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)benzol; p-Toluylendiamin / 2-Amino-3-hydroxypyridin; p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diaminophenoxy)ethanol; 2-(2-Hydroxyethyl)-p-phenylendiamin / 1,3-Bis(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxypyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1H-imidazol-1-yl)propyl]amin / 5-Amino-2-methylphenol; N-(4-Amino-3-methylphenyl)-N-[3-(1H-imidazol-1-yl)propyl]amin / 3-Aminophenol; N-(4-Amino-3-methylphenyl)-N-[3-(1H-imidazol-1-yl)propyl]amin / 2-(2,4-Diaminophenoxy)ethanol; N-(4-Amino-3-methylphenyl)-N-[3-(1H-imidazol-1-yl)propyl]amin / 1,3-Bis(2,4-diaminophenoxy)-propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)pyrazol / Resorcin; 4,5-Diamino-1-(2-hydroxyethyl)pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diaminophenoxy)ethanol; 4,5-Diamino-1-(2-hydroxyethyl)pyrazol / 1,3-Bis(2,4-diaminophenoxy)-propan; 4,5-Diamino-1-(2-hydroxyethyl)pyrazol / 1-Methoxy-2-amino-4-(2-hydroxyethylamino)-benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

Um eine ausgewogene und subtile Nuancenausbildung zu erzielen, ist es erfindungsgemäß vorteilhaft, wenn weitere farbgebende Komponenten in dem Mittel enthalten sind, das in Schritt b) dieser Variante des erfindungsgemäßen Verfahrens eingesetzt wird.

In einer weiteren Ausführungsform können die in Schritt b) dieser Variante des erfindungsgemäßen Verfahrens eingesetzten Mittel zusätzlich mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die erfindungsgemäße Vorgehensweise führt zu einer Verringerung oder Verhinderung einer schädigenden Hydrophilierung der Haaroberfläche - das Haar bleibt geschmeidig und hydrophil, weist ein natürliches Haargefühl auf und läßt sich dadurch gut kämmen und frisieren. Darüber hinaus wird die Haarschädigung aufgrund oxidativer Haarbehandlung deutlich minimiert.

Weitere erfindungsgemäße Gegenstände sind demnach ein Verfahren zur Verringerung oder Verhinderung einer schädigenden Hydrophilierung der Haaroberfläche durch oxidative Haarbehandlung, bei dem vor der oxidativen Haarbehandlung ein Vorbehandlungsmittel, enthaltend aminofunktionelle(s) Silikon(e) mit terminale(r/n) Hydroxygruppe(n) auf die keratinischen Fasern aufgetragen wird sowie ein Verfahren zur Verringerung oder Verhinderung der Haarschädigung aufgrund oxidativer Haarbehandlung, bei dem vor der oxidativen Haarbehandlung ein Vorbehandlungsmittel, enthaltend aminofunktionelle(s) Silikon(e) mit terminale(r/n) Hydroxygruppe(n) auf die keratinischen Fasern aufgetragen wird und ein Verfahren zur Erzeugung einer waschbeständigen Pflegewirkung vor einer oxidativen Haarbehandlung, bei dem vor der oxidativen Haarbehandlung ein Vorbehandlungsmittel, enthaltend aminofunktionelle(s) Silikon(e) mit terminale(r/n) Hydroxygruppe(n) auf die keratinischen Fasern aufgetragen wird.

Für bevorzugte Ausführungsformen dieser drei erfindungsgemäßen Verfahren gilt mutatis mutandis das zum ersten erfindungsgemäßen Verfahren Gesagte.

Die folgenden Beispiele sollen den Gegenstand der Erfindung weiter verdeutlichen.

### Beispiele:

Es wurden drei Vorbehandlungsmittel, zwei Blondiermittel und zwei Haarfärbemittel bereitgestellt.

Alle Vorbehandlungsmittel V1, V2 und V3 enthielten jeweils 0,2 Gew.-% eines OH-terminierten Aminosilikons und waren bis auf ihren Gehalt an Polyacrylsäure identisch zusammengesetzt.

Das Vorbehandlungsmittel V1 (erfindungsgemäß) wies bei Normalbedingungen (20°C, 1013,25 mbar) eine Viskosität (Brookfield RTV, Spindel 4, 20 U/min) von 800 mPas auf. Das Vorbehandlungsmittel V2 (nicht erfindungsgemäß) enthielt kein Carbomer und wies bei Normalbedingungen (20°C, 1013,25 mbar) eine Viskosität (Brookfield RTV, Spindel 4, 20 U/min) von 0,8 mPas auf. Das Vorbehandlungsmittel V3 (nicht erfindungsgemäß) enthielt mehr Carbomer und wies bei Normalbedingungen (20°C, 1013,25 mbar) eine Viskosität (Brookfield RTV, Spindel 4, 20 U/min) von 4000 mPas auf.

Auch die zwei Blondiermittel und zwei Haarfärbemittel waren jeweils bis auf den Gehalt an Wasserstoffperoxid identisch zusammengesetzt und enthielten (B1 bzw. H1, jeweils erfindungsgemäß) 4 Gew.-% Wasserstoffperoxid, berechnet als 100%iges H₂O₂ bzw. (B2 bzw. H2, jeweils nicht erfindungsgemäß) 2 Gew.-% Wasserstoffperoxid, berechnet als 100%iges H₂O₂.

Mit den Kombinationen der jeweiligen Mittel wurden Keratinfasern erst vor- und dann oxidatiov behandelt:
- V1 → B1: erfindungsgemäßes Verfahren
- V1 → H1: erfindungsgemäßes Verfahren
- V2→ B1: nicht erfindungsgemäß (zu niedrige Viskosität des Vorbehandlungsmittels)
- V2→ H1: nicht erfindungsgemäß (zu niedrige Viskosität des Vorbehandlungsmittels)
- V3→ B1: nicht erfindungsgemäß (zu hohe Viskosität des Vorbehandlungsmittels)
- V3→ H1: nicht erfindungsgemäß (zu hohe Viskosität des Vorbehandlungsmittels)
- V1 → B2: nicht erfindungsgemäßes Verfahren (zu wenig H₂O₂ im oxidativen Mittel)
- V1 → H2: erfindungsgemäßes Verfahren (zu wenig H₂O₂ im oxidativen Mittel)
- V2 → B2: nicht erfindungsgemäß (zu niedrige Viskosität des Vorbehandlungsmittels und zu wenig H₂O₂ im oxidativen Mittel)
- V2 → H2: nicht erfindungsgemäß (zu niedrige Viskosität des Vorbehandlungsmittels und zu wenig H₂O₂ im oxidativen Mittel)
- V3 → B2: nicht erfindungsgemäß (zu hohe Viskosität des Vorbehandlungsmittels und zu wenig H₂O₂ im oxidativen Mittel)
- V3 → H2: nicht erfindungsgemäß (zu hohe Viskosität des Vorbehandlungsmittels und zu wenig H₂O₂ im oxidativen Mittel)

Die Ergebnisse der zwölf unterschiedlichen Verfahrensvarianten im Hinblick auf den Faserschutz und die Qualität der oxidativen Behandlung zeigt die nachfolgende Tabelle. Dabei wurde der Faserschutz mit Kontaktwinkelmessungen bestimmt, die Beurteilung der Blondier- bzw. Färbeergebnisse erfolgte durch ein Expertenpanel aus 10 Experten.

| **Verfahren** | **Faserschutz** | **Ergebnis der oxidativen Behandlung** |
|---|---|---|
| V1 → B1 | +++ | gleichmäßige Blondierung |
| V1 → H1 | +++ | gleichmäßige Färbung |
| V2 → B1 | - | gleichmäßige Blondierung |
| V2→ H1 | - | gleichmäßige Färbung |
| V3→ B1 | - | ungleichmäßige Blondierung, fleckiges Erscheinungsbild |
| V3→ H1 | - | ungleichmäßige Färbung, fleckiges Erscheinungsbild |
| V1 → B2 | ++ | schlechtes Blondierergebnis, keine ausreichende Aufhellung, ungleichmäßige Aufhellung |
| V1 → H2 | ++ | schlechtes Färbeergebnis, keine ausreichende Färbung, ungleichmäßige Färbung |
| V2 → B2 | - | sehr schlechtes Blondierergebnis, keine ausreichende Aufhellung, sehr ungleichmäßige Aufhellung |
| V2 → H2 | - | sehr schlechtes Färbeergebnis, keine ausreichende Färbung, sehr ungleichmäßige Färbung |
| V3 → B2 | - | sehr schlechtes Blondierergebnis, keine ausreichende Aufhellung, sehr ungleichmäßige Aufhellung |
| V3→ H2 | - | sehr schlechtes Färbeergebnis, keine ausreichende Färbung, sehr ungleichmäßige Färbung |

Die Ergebnisse zeigen, daß nur die erfindungsgemäßen Verfahrensvarianten gute Ergebnisse mit hervorragendem Faserschutz vereinen.

## Patentansprüche

1. Verfahren zur oxidativen Haarbehandlung, bei dem
a) ein Vorbehandlungsmittel, enthaltend aminofunktionelle(s) Silikon(e) mit terminale(r/n) Hydroxygruppe(n) auf die keratinischen Fasern aufgetragen wird, wobei das Vorbehandlungsmittel,
a als Spray aufgesprüht oder
b als Gel mit einer Viskosität unter Normalbedingungen (20°C und 1012,25 mbar) von 500 bis 5000 mPas (Brookfield RTV, Spindel 4, 20 U/min) aufgetragen wird oder
c als Spülung mit einer Viskosität unter Normalbedingungen (20°C und 1012,25 mbar) kleiner 500 mPas (Brookfield RTV, Spindel 4, 20 U/min) aufgetragen wird,
b) die keratinischen Fasern innerhalb eines Zeitraumes von einer Sekunde bis 24 Stunden nach Schritt a) mit einem oxidativen Haarbehandlungsmittel behandelt werden, wobei
- das oxidative Haarbehandlungsmittel eine pH Wert > 8,5 aufweist
- das oxidative Haarbehandlungsmittel bezogen auf sein Gewicht mindestens 3 Gew.-% Wasserstoffperoxid (berechnet als 100%iges H₂O₂) enthält.

2. Verfahren nach Anspruch 1, **dadurch gekennzeichnet, daß** das Vorbehandlungsmittel bezogen auf sein Gewicht 0,00001 bis 10 Gew.-%, vorzugsweise 0,0001 bis 7,5 Gew.-%, weiter bevorzugt 0,001 bis 5 Gew.-%, noch weiter bevorzugt 0,01 bis 2,5 Gew.-% und insbesondere 0,1 bis 1 Gew.-% aminofunktionelle(s) Silikon(e) mit terminale(r/n) Hydroxygruppee(n) enthält.

3. Verfahren nach einem der Ansprüche 1 oder 2, **dadurch gekennzeichnet, daß** das Vorbehandlungsmittel frei ist von Silikonen der Formeln (I) und (II) 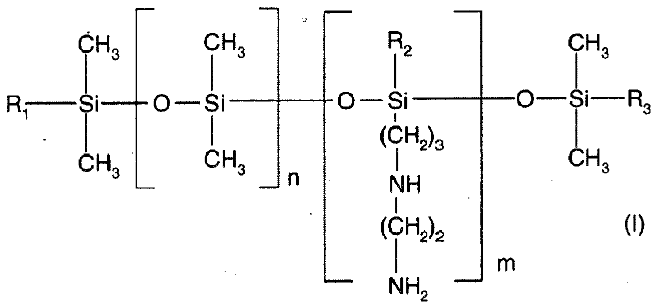 in der Formel (I):
- m und n bedeuten Zahlen, die so gewählt sind, dass die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Alkoxygruppe bedeutet; 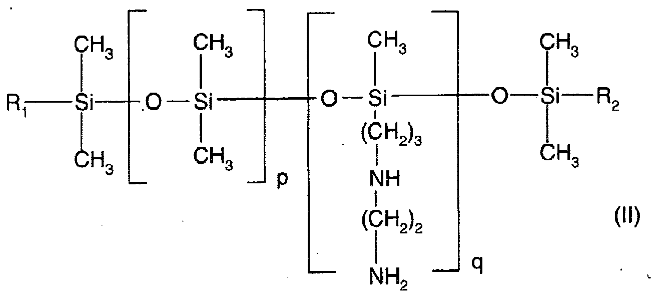
in der Formel (II):
- p und q bedeuten Zahlen, die so gewählt sind, dass die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Alkoxygruppe bedeutet.

4. Verfahren nach einem der Ansprüche 1 bis 3, **dadurch gekennzeichnet, daß** das Vorbehandlungsmittel mindestens ein Silikon der Formel (III) enthält: 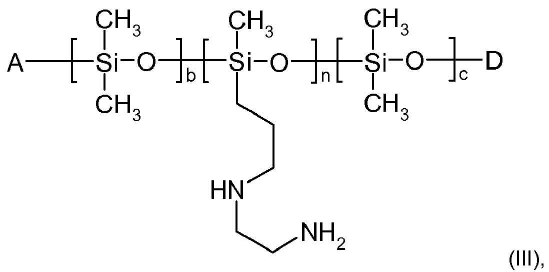 in der
A für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
b, n und c für ganze Zahlen zwischen 0 und 1000 stehen,
mit den Maßgaben
- n > 0 und b + c > 0
- mindestens eine der Bedingungen A = -OH bzw. D = -H ist erfüllt.

5. Verfahren nach einem der Ansprüche 1 bis 4, **dadurch gekennzeichnet, daß** das Vorbehandlungsmittel mindestens ein 4-morpholinomethyl-substituiertes Silikon der Formel (IV) 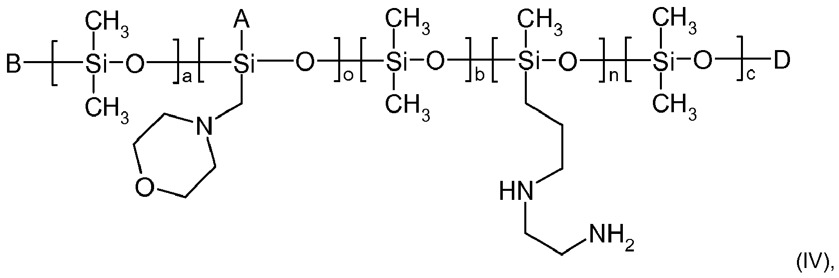 in der
A für eine über ein -O- gebundene Struktureinheit (i), (ii) oder (iii) 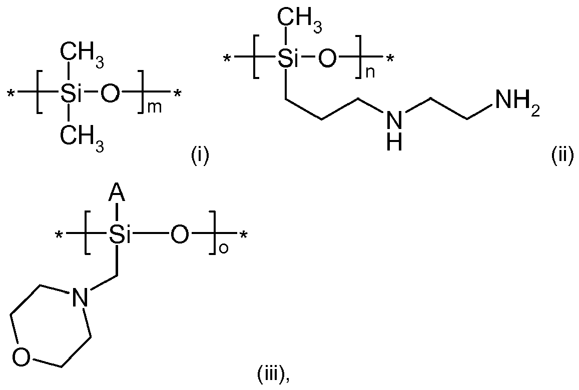 oder einen über ein -O- gebundenen oligomeren oder polymeren Rest enthaltend Struktureinheiten der Formeln (I), (II) oder (III) oder die Hälfte eines verbindenden O-Atoms zu einer Struktureinheit (III) oder für-OH steht,
* für eine Bindung zu einer der Struktureinheiten (i), (ii) oder (iii) oder für eine Endgruppe B (Si-gebunden) oder D (O-gebunden) steht,
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0 m, n und o für ganze Zahlen zwischen 1 und 1000 stehen,
mit der Maßgabe, daß mindestens eine der Bedingungen B = -OH bzw. D = -H erfüllt ist.

6. Verfahren nach einem der Ansprüche 1 bis 5, **dadurch gekennzeichnet, daß** das Vorbehandlungsmittel mindestens ein 4-morpholinomethyl-substituiertes Silikon der Formeln (IVa), (IVb), (IVc), (IVd), (IVe) oder (IVf) 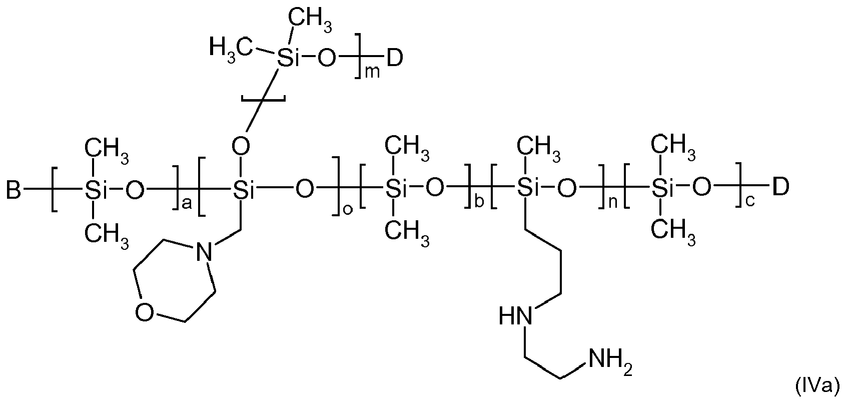 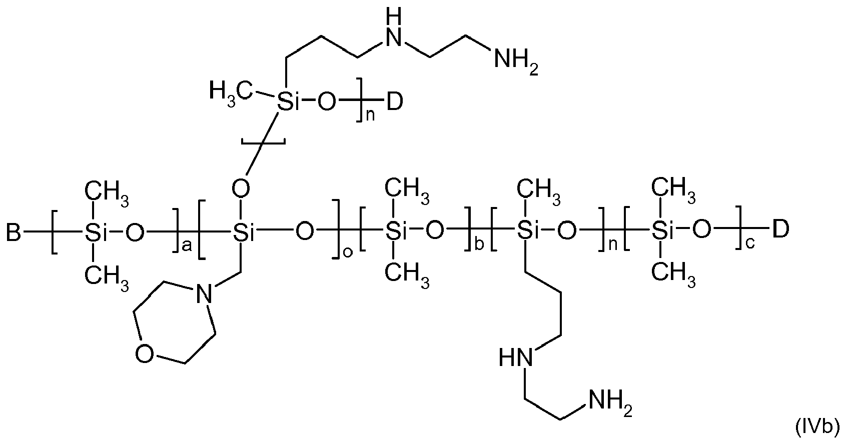 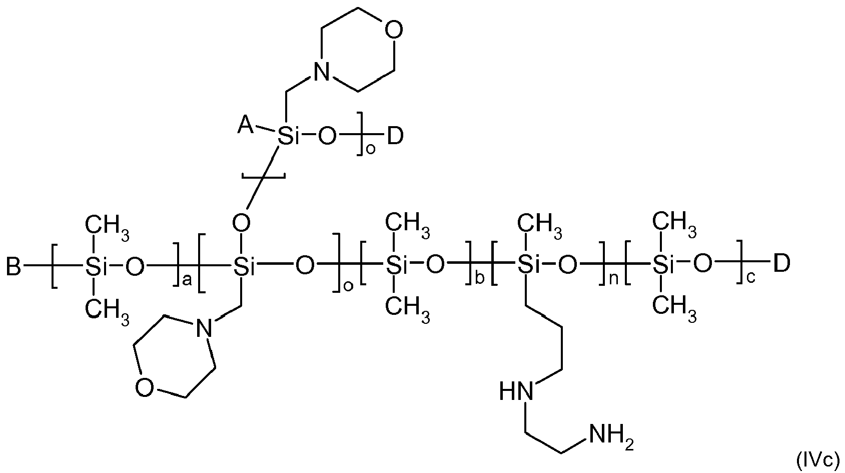 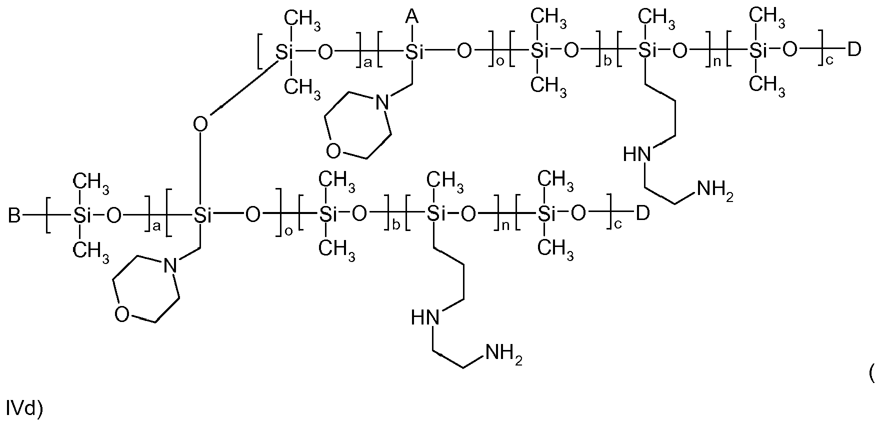 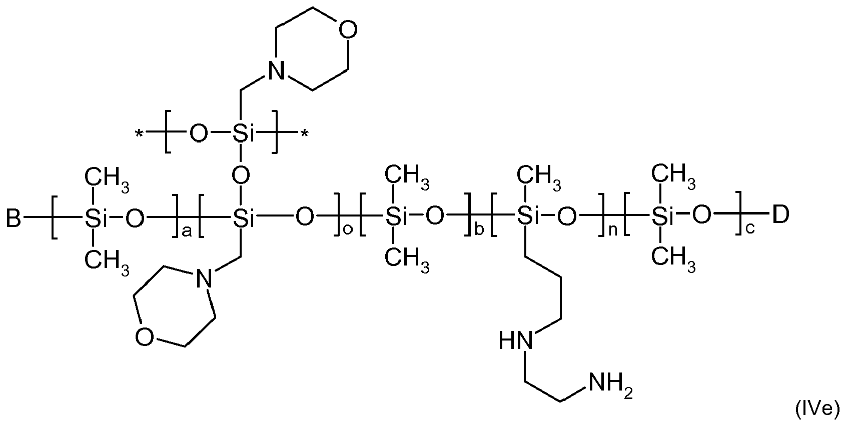 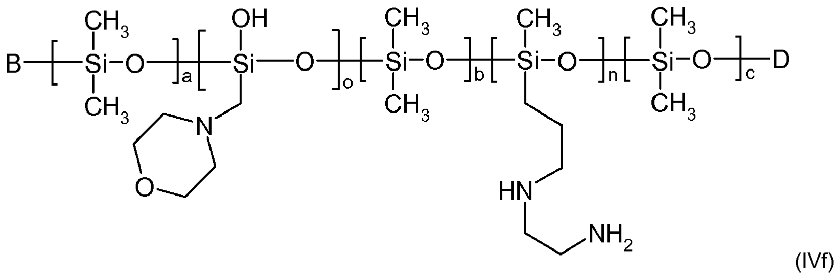 enthält, in denen
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
a, b und c für ganze Zahlen zwischen 0 und 1000 stehen, mit der Maßgabe a + b + c > 0
m, n und o für ganze Zahlen zwischen 1 und 1000 stehen,
mit der Maßgabe, daß mindestens eine der Bedingungen B = -OH bzw. D = -H erfüllt ist.

7. Verfahren nach einem der Ansprüche 1 bis 6, **dadurch gekennzeichnet, daß** das Vorbehandlungsmittel - bezogen auf sein Gewicht - 0,00001 bis 10 Gew.-%, vorzugsweise 0,0001 bis 7,5 Gew.-%, besonders bevorzugt 0,001 bis 5 Gew.-%, weiter bevorzugt 0,01 bis 3 Gew.-% und insbesondere 0,1 bis 1 Gew.-% 4-morpholinomethyl-substituierte(s) Silikon(e) enthält.

8. Verfahren nach einem der Ansprüche 1 bis 7, **dadurch gekennzeichnet, daß** das Vorbehandlungsmittel in Form einer Mikroemulsion vorliegt, welche Fettalkohol(e) enthält.

9. Verfahren nach einem der Ansprüche 1 bis 8, **dadurch gekennzeichnet, daß** das Vorbehandlungsmittel - bezogen auf sein Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) enthält.

10. Verfahren nach einem der Ansprüche 1 bis 9, **dadurch gekennzeichnet, daß** das Vorbehandlungsmittel - bezogen auf sein Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% α-iso-Tridecyl-ω-hydroxypolyglycolether (INCI-Bezeichnung: Trideceth-10) enthält.

11. Verfahren nach einem der Ansprüche 1 bis 10, **dadurch gekennzeichnet, daß** das Vorbehandlungsmittel - bezogen auf sein Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% einer Mischung aus verzweigtem, ethoxyliertem Tridecanol (INCI-Bezeichnung: Trideceth-5) und α-iso-Tridecyl-ω-hydroxypolyglycolether (INCI-Bezeichnung: Trideceth-10) enthält.

12. Verfahren zur Verringerung oder Verhinderung einer schädigenden Hydrophilierung der Haaroberfläche durch oxidative Haarbehandlung, **dadurch gekennzeichnet, daß** vor der oxidativen Haarbehandlung ein Vorbehandlungsmittel, enthaltend aminofunktionelle(s) Silikon(e) mit terminale(r/n) Hydroxygruppe(n) auf die keratinischen Fasern aufgetragen wird.

13. Verfahren zur Verringerung oder Verhinderung der Haarschädigung aufgrund oxidativer Haarbehandlung, **dadurch gekennzeichnet, daß** vor der oxidativen Haarbehandlung ein Vorbehandlungsmittel, enthaltend aminofunktionelle(s) Silikon(e) mit terminale(r/n) Hydroxygruppe(n) auf die keratinischen Fasern aufgetragen wird.

14. Verfahren zur Erzeugung einer waschbeständigen Pflegewirkung vor einer oxidativen Haarbehandlung, **dadurch gekennzeichnet, daß** vor der oxidativen Haarbehandlung ein Vorbehandlungsmittel, enthaltend aminofunktionelle(s) Silikon(e) mit terminale(r/n) Hydroxygruppe(n) auf die keratinischen Fasern aufgetragen wird.
